(19) Europäisches Patentamt
European Patent Office
Office européen des brevets

(11) **EP 4 129 156 A1**

(12) **EUROPEAN PATENT APPLICATION**
published in accordance with Art. 153(4) EPC

(43) Date of publication:
**08.02.2023 Bulletin 2023/06**

(21) Application number: **20926629.5**

(22) Date of filing: **24.03.2020**

(51) International Patent Classification (IPC):
***A61B 3/10*** (1968.09)

(52) Cooperative Patent Classification (CPC):
**A61B 3/102; A61B 3/10**

(86) International application number:
**PCT/JP2020/013050**

(87) International publication number:
**WO 2021/192047 (30.09.2021 Gazette 2021/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **NEC Corporation
108-8001 Tokyo (JP)**

(72) Inventor: **NAKAMURA, Shigeru
Tokyo 108-8001 (JP)**

(74) Representative: **Betten & Resch
Patent- und Rechtsanwälte PartGmbB
Maximiliansplatz 14
80333 München (DE)**

(54) **OPTICAL COHERENCE TOMOGRAPHY DEVICE, IMAGING METHOD, AND NON-TRANSITORY COMPUTER-READABLE MEDIUM IN WHICH IMAGING PROGRAM IS STORED**

(57) The optical coherence tomography apparatus (100) includes a splitter/merger (104), which splits an object light beam, which is split from light emitted from a wavelength sweeping laser light source (101) at a splitter/merger (103), into a plurality of object light beams, an optical spectrum data generation unit (110), which generates information on wavelength dependence of the intensity difference among a plurality of interference light beams generated by an interference between an object light beam and a reference light beam at the splitter/merger (103), wherein the object light beam is generated by merging the plurality of object light beams one another at the splitter/merger (104) after the plurality of object light beams are radiated to different positions on the surface of a measurement target object (200) and then scattered by the measurement target object (200), and a control unit (111), which acquires structural data in the depth direction of the measurement target object (200) based on information on wavelength dependence of the intensity difference among the plurality of interference light beams and connects a plurality of sets of acquired structural data in the depth direction to each other while moving the radiation position of the plurality of object light beams along the direction in which the measurement target object (200) is scanned.

Fig. 1

**Description**

**Technical Field**

The present invention relates to an optical coherence tomography apparatus, an imaging method, and a non-transitory computer readable medium storing an imaging program.

**Background Art**

As a technique for performing tomographic imaging in the vicinity of a surface of a measurement object, there is an optical coherence tomography (OCT) technique. In the OCT technique, tomographic imaging in the vicinity of a surface of a measurement target object is performed when the measurement target object is irradiated with a light beam and by utilizing interference between a scattered light beam (hereinafter also referred to as a "backscattered light beam") from the interior of the measurement target object and a reference light beam. In recent years, the OCT technique has been increasingly applied to medical diagnosis and industrial product inspection.

The OCT technique uses interference between an object light beam radiated onto and scattered by the measurement target object and scattered therefrom and the reference light to identify the position in the optical axis direction, that is, the depth direction, of a portion of the measurement target object where the object light beam is scattered (light scattering point). Structural data spatially resolved in the depth direction of the measurement target object is thus generated. In many cases, the object light beam is not reflected by 100% only off the surface of the measurement target object, but propagates to some extent into the measurement target object and is then scattered backward. Structural data spatially resolved in the depth direction on the interior of the measurement target object can thus be generated. The OCT technique is classified into a time domain (TD-OCT) scheme and a Fourier domain (FD-OCT) scheme, and the FD-OCT scheme is more promising in terms of high speed and high sensitivity. In the FD-OCT scheme, when the object light beam interferes with the reference light beam, an interference light beam spectrum over a wide wavelength band is measured, and the spectrum is Fourier transformed into structural data in the depth direction. There are two schemes for generating an interference light beam spectrum, a spectral domain (SD-OCT) scheme using a spectrometer, and a swept source (SS-OCT) scheme using a wavelength-sweeping light source.

Furthermore, scanning the measurement target object with the object light beam in the in-plane direction perpendicular to the depth direction of the measurement target object allows generation of tomographic structural data spatially resolved in the in-plane direction and the depth direction. Three-dimensional tomographic structural data on the measurement target object can thus be generated. A galvanometric mirror or any other similar device usually sweeps the object light beam to move the radiation position of the single object light beam.

The OCT technique has been put into practical use as a fundus tomography apparatus in ophthalmological diagnosis, and the application of the OCT technique to a noninvasive tomography apparatus for a variety of biological sites is being studied.

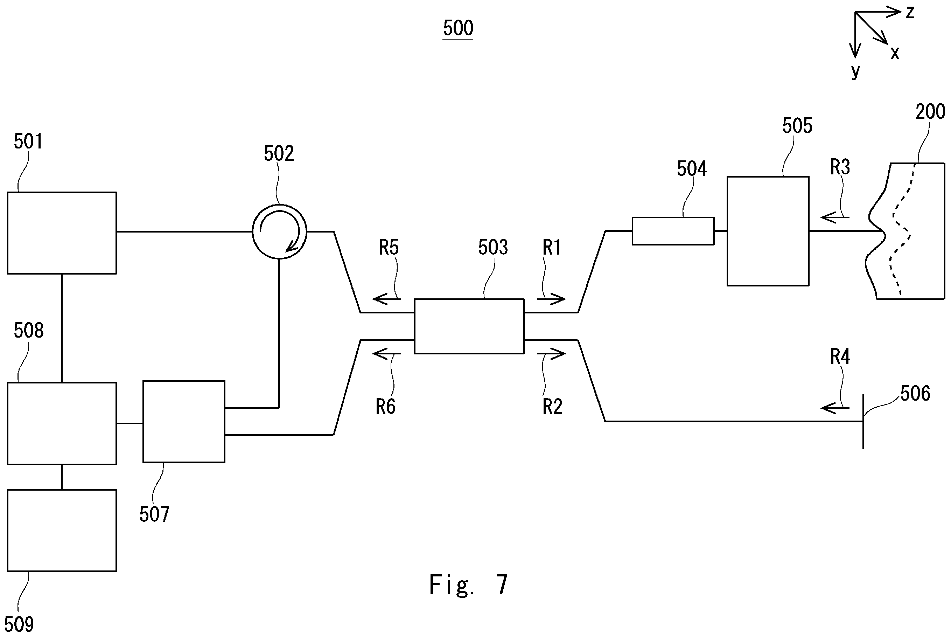

Fig. 7 shows a typical configuration of an optical coherence tomography apparatus 500 based on the SS-OCT scheme. A wavelength-sweeping laser light source 501 generates wavelength-swept optical pulses. The light beam emitted from the laser light source 501 is split at a splitter/merger 503 via a circulator 502 into an object light beam R1 and a reference light beam R2. The object light beam R1 is radiated onto a measurement target object 200 via a fiber collimator 504 and a radiation optical system 505 formed of a scan mirror and a lens. An object light beam R3 scattered in the measurement target object 200 then returns to the splitter/merger 503. On the other hand, the reference light beam R2 returns to the splitter/merger 503 via a reference light beam mirror 506. The splitter/merger 503, in which the object light beam R3 scattered from the measurement target object 200 interferes with the reference light beam R4 reflected off the reference light beam mirror 506, therefore generates interference light beams R5 and R6. The phase difference between the object light beam R3 and the reference light beam R4 therefore determines the intensity ratio between the interference light beam R5 and the interference light beam R6. The interference light beam R5 is input to a two-input balanced light receiver 507 via the circulator 502, and the interference light beam R6 is directly input the two-input balanced light receiver 507.

The intensity ratio between the interference light beam R5 and the interference light beam R6 changes as the wavelength of the light emitted from the wavelength-sweeping laser light source 501 changes. The photoelectrically converted output from the balanced light receiver 507 can thus be measured as an interference light beam spectrum. Measuring and Fourier transforming the interference light beam spectrum allows generation of data representing the intensity of the backscattered light beam (object light beam) at different positions in the depth direction (direction Z) (operation of generating data representing the intensity of the backscattered light beam (object light beam) in the depth direction (direction Z) at a certain position in the measurement target object 200 is hereinafter referred to as "A scan").

Consider now the interference between an object light beam and a reference light beam having a wavelength

$\lambda$ and a wave number k (=$2\pi/\lambda$). Let $L_R$ be the optical path length from the point where the reference light beam is split by the splitter/merger 503 to the point where the reference light beam is reflected off the reference light beam mirror 506 and returns to the splitter/merger 503, and $L_S$=$L_R$+$z_0$ be the optical path length from the point where the object light beam is split by the splitter/merger 503 to the point where the object light beam is backscattered at a single light scattering point in the measurement target object 200 and returns to the splitter/merger 503, the object light beam R3 and the reference light beam R4 interfere with each other at the splitter/merger 503 with a phase difference $kz_0$+$\varphi$ therebetween. The symbol $\varphi$ is a constant independent of k and $z_0$. Let $E_S$ be the amplitude of the object light beam R3, which undergoes the interference at the splitter/merger 503, and $E_R$ be the amplitude of the reference light beam R4, the intensity difference between the interference light beam R5 and the interference light beam R6, which is expressed by the following expression, is photoelectrically converted by the balanced light receiver 507.

[Expression 1]

$$I(k) \propto E_S \cdot E_R \cdot \cos(kz_0 + \phi)$$

An optical spectrum data generation unit 508 generates interference light beam spectrum data based on information on a change in the wavelength of the light emitted from the wavelength-sweeping laser 501 and information on the intensity difference between the interference light beam R5 and the interference light beam R6 from the balanced light receiver 507. Interference light beam spectrum data I(k) generated by the measurement made from a wave number $k_0$-$\Delta k/2$ to $k_0$+$\Delta k/2$ shows modulation with a cycle $2\pi/z_0$. The generated interference light beam spectrum data I(k) is sent from the optical spectrum data generation unit 508 to a control unit 509. Note that $k_0$ satisfies $k_0$=$2\times\pi$/(wavelength-sweeping light source center wavelength).

The control unit 509 performs Fourier transformation on the interference light beam spectrum data I(k). An amplitude J(z) generated as a result of the Fourier transformation of the interference light beam spectrum data I(k) is expressed by the following expression:

[Expression 2]

$$J(z) = \left| \int I(k)e^{izk}dk \right| \propto \delta(z - z_0) + \delta(z + z_0)$$

The amplitude J(z) represents a peak expressed as a $\delta$ function (delta function) (hereafter referred to as "$\delta$-function-like peak") at z=$z_0$ (and z=-$z_0$), which reflects a light scattering position $z_0$. Although a mirror has one light scattering point, the object light beam radiated onto the measurement target object 200 is usually backscattered successively as the object light beam propagates to some extent into the measurement target object 200 while being attenuated, so that the light scattering points, at which the object light beam is scattered, are distributed over a range from the surface of the measurement target object 200 to a certain depth. When the light scattering points are distributed from $z_0$-$\Delta z$ to $z_0$+$\Delta z$ in the depth direction, an interference light beam spectrum shows modulation with a period $2\pi/(z_0-\Delta z)$ to modulation with $2\pi/(z_0+\Delta z)$, which overlap with each other.

The radiation position of the object light beam R1 is moved by the radiation optical system 505, so that the measurement target object 200 is scanned. The radiation optical system 505 repeatedly performs the A-scan operation while moving the radiation position of the object light beam R1 in a scanning line direction (direction X) and connecting the measurement results to each other, thereby generating a map of the intensities of two-dimensional backscattered light beams (object light beams) in the scanning line direction and the depth direction as the tomographic structural data (operation of repeatedly performing the A-scan operation in the scanning line direction (direction X) and connecting the measurement results to each other is hereinafter referred to as "B Scan").

Furthermore, the radiation optical system 505 repeatedly performs the B-scan operation while moving the radiation position of the object light beam R1 not only in the scanning line direction but also in a direction (direction Y) perpendicular to the scanning line, and connects the measurement results to each other, thereby generating three-dimensional tomographic structural data (operation of repeatedly performing the B-scan operation in the direction (direction Y) perpendicular to the scanning line and connecting the measurement results to each other is hereinafter referred to as "C Scan").

When the measurement target object is a living body, it is usually difficult to measure the living body with the living body completely immobilized. It is therefore necessary to make the measurement at high speed. When the measurement needs to be made over a wide range, it is difficult to make the measurement over the wide range at high speed simply by scanning the range with a single object light beam at high speed. The Patent Literature 1 therefore proposes a configuration in which a plurality of object light beams are radiated.

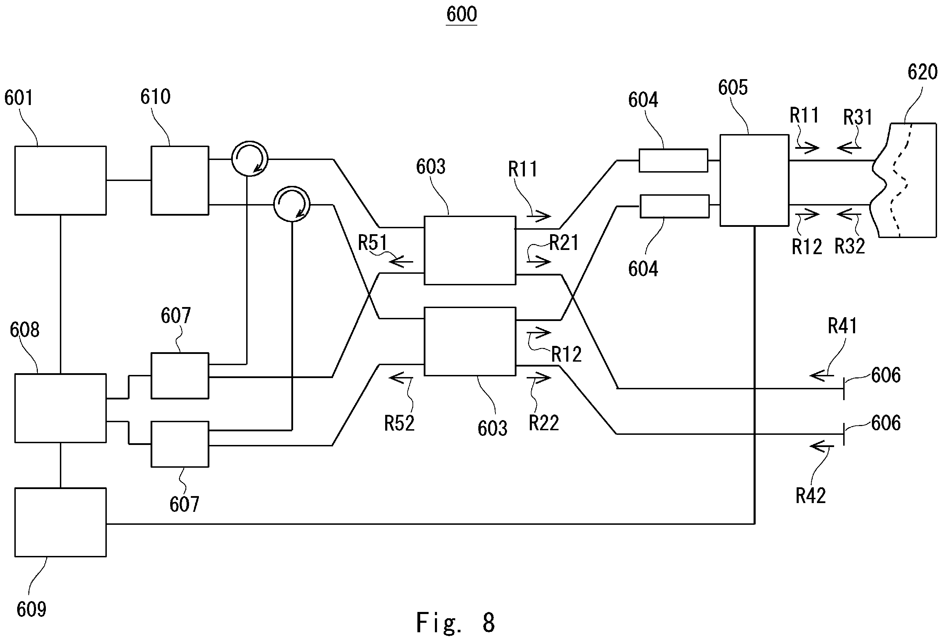

Fig. 8 shows a typical configuration of an optical coherence tomography apparatus 600 based on the SD-OCT scheme, in which a plurality of object light beams are radiated, as described in Patent Literature 1. A light source 601 emits light having a wide wavelength range of about 100 nm. The light emitted from the light source 601 is split by a splitter 610 into N split light beams (N=2 in Fig. 8), and then divided into the object light beam R1 and the reference light beam R2 by a plurality of splitters/mergers 603. A plurality of object light beams R11 and R12 are radiated onto the measurement target object 200 via a plurality of collimators 604 and an optical system 605 formed of a scan mirror and a lens. In the description, two object light beams R11 and R12 are simultaneously radiated to different positions on the measurement target object 200, and backscattered light beams R31 and R32 return to the splitters/mergers 603. On the other hand, the plurality of reference light beams R21 and R22 are reflected off a plurality of reference light beam mirrors 606 and return to the splitters/mergers 603. At the plurality of splitters/mergers 603, the object light beams R31 and R32 scattered by the measurement target object 200 interfere with the reference light beams R41 and R42 reflected off the mirrors. A plurality of spectrometers 607 generate the spectra of the interference light beams R51 and R52 to generate structural data in the depth direction at the object light radiation position on the measurement target object 200.

A plurality of different areas of the measurement target object 200 are simultaneously scanned with the plurality of object light beams R11 and R12, whereby high-speed measurement over a wide range is achieved. On the other hand, it is necessary to provide a plurality of reference optical paths and a plurality of photodetectors or spectrometers corresponding to the object light beams R11 and R12.

Patent Literature 2 describes a configuration in which a plurality of object light beams are radiated to achieve high spatial resolution. Also in this case, a plurality of photodetectors are required although the reference optical path corresponding to the plurality of object light beams is shared.

## Citation List

### Patent Literature

Patent Literature 1 Japanese Unexamined Patent Application Publication No. 2010-167253

Patent Literature 2 International Publication WO2006/054116

## Summary of Invention

### Technical Problem

The aforementioned optical coherence tomography apparatus, which radiates a plurality of object light beams, requires a plurality of reference optical paths and a plurality of light receivers, resulting in an increase in size and cost as compared with the case where a single object light beam is radiated.

An object of the present invention is to provide a compact optical coherence tomography apparatus, an imaging method, and a non-transitory computer readable medium storing an imaging program each capable of wide-range, high-speed measurement at low cost.

### Solution to Problem

An optical coherence tomography apparatus according to a first aspect of the present invention includes a wavelength sweeping laser light source, a first splitter unit that splits a light beam emitted from the wavelength sweeping laser light source into an object light beam and a reference light beam, a second splitter unit that splits the object light beam output from the first splitter unit into a plurality of object light beams, a radiating unit that radiates the plurality of object light beams output from the second splitter unit to different positions on a surface of a measurement target object, a merging unit that causes an object light beam to interfere with the reference light beam to generate a plurality of interference light beams, the object light beam generated by merging the plurality of object light beams obtained by radiation onto the measurement target object and then scattering by the measurement target object, with one another at the second splitter unit, a measuring unit that generates information on wavelength dependence of an intensity difference among the plurality of interference light beams output from the merging unit, and a control unit that acquires structural data in a depth direction of the measurement target object based on the information relating to wavelength dependence of an intensity difference among the plurality of interference beams and generated by the measuring unit, acquires a plurality of sets of structural data in the depth direction while controlling the radiating unit to move radiation positions of the plurality of object light beams along a direction perpendicular to the depth direction of the measurement target object, and connects the plurality of sets of acquired structural data in the depth direction to each other.

An imaging method according to a second aspect of the present invention includes causing a control unit to, after an object light beam split from a light beam emitted from a wavelength sweeping laser light source is further split into a plurality of object light beams, and the plurality of object light beams are radiated to different positions on a target object, then scattered by the measurement target object, and merged with one another into an object light beam, acquire structural data in a depth direction of a measurement target object based on information on wavelength dependence of an intensity difference among a plurality of interference light beams generated when the object light beam interferes with a reference light beam split from the light beam emitted from the wavelength sweeping laser light source, causing the control unit to acquire a plurality of sets of structural data in the depth direction while moving radiation positions of the plurality of object light beams along a direction perpendicular to the depth direction of the measurement target object, and causing the control unit to connect the plurality of sets of acquired structural data in the depth direction to each other.

A non-transitory computer readable medium storing an imaging program according to a third aspect of the present invention causes a control unit to, after an object light beam split from a light beam emitted from a wavelength sweeping laser light source is further split into a plurality of object light beams, and the plurality of object light beams are radiated to different positions on a target object, then scattered by the measurement target object, and merged with one another into an object light beam, execute a process of acquiring structural data in a depth direction of a measurement target object based on information on wavelength dependence of an intensity difference among a plurality of interference light beams generated when the object light beam interferes with a reference light beam split from the light beam emitted from the wavelength sweeping laser light source, a process of acquiring a plurality of sets of structural data in the depth direction while moving radiation positions of the plurality of object light beams along a direction perpendicular to the depth direction of the measurement target object, and a process of connecting the plurality of sets of acquired structural data in the depth direction to each other.

**Advantageous Effects of Invention**

A compact optical coherence tomography apparatus, an imaging method, and a non-transitory computer readable medium storing an imaging program each capable of wide-range, high-speed measurement at low cost can be provided.

**Brief Description of Drawings**

Fig. 1 is a block diagram showing an example of an optical coherence tomography apparatus according to the present invention;
Fig. 2 shows an example of the optical coherence tomography apparatus according to a first example embodiment of the present invention;
Fig. 3A describes data generated in A scan in the optical coherence tomography apparatus according to the first example embodiment of the present invention;
Fig. 3B describes data generated in the A scan in the optical coherence tomography apparatus according to the first example embodiment of the present invention;
Fig. 3C describes data generated in B scan in the optical coherence tomography apparatus according to the first example embodiment of the present invention;
Fig. 3D describes data generated in the B scan in the optical coherence tomography apparatus according to the first example embodiment of the present invention;
Fig. 4 shows an example of the optical coherence tomography apparatus according to a second example embodiment of the present invention;
Fig. 5 shows an example of the optical coherence tomography apparatus according to a third example embodiment of the present invention;
Fig. 6 shows an example of the optical coherence tomography apparatus according to a fourth example embodiment of the present invention;
Fig. 7 shows an example of the optical coherence tomography apparatus relating to the present invention; and
Fig. 8 shows another example of the optical coherence tomography apparatus relating to the present invention.

## Example Embodiment

Example embodiments of the present invention will be described below with reference to the drawings.

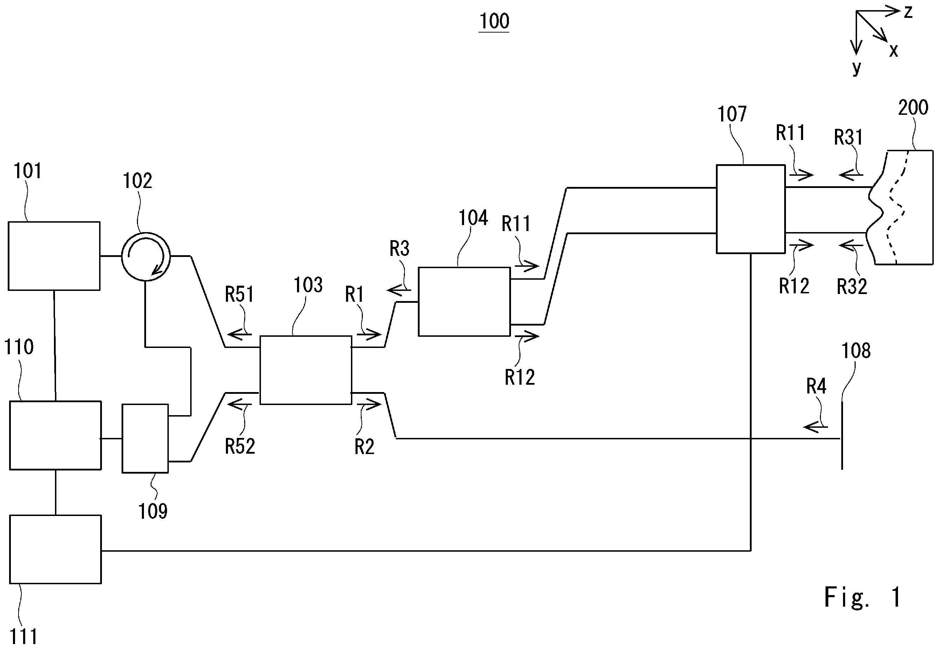

Fig. 1 is a block diagram showing an example of an optical coherence tomography apparatus 100 according to the present invention. The optical coherence tomography apparatus 100 includes a wavelength sweeping laser light source 101, a circulator 102, a splitter/merger 103, which serves as a first splitter unit and merging unit, a splitter/merger 104, which serves as a second splitter unit, a radiation optical system 107, which serves as a radiating unit, a reference light beam mirror 108, a balanced light receiver 109, which serves as a measuring unit, an optical spectrum data generation unit 110, which serves as the measuring unit, a control unit 111, and other components, as shown in Fig. 1.

The light emitted from the wavelength sweeping laser light source 101 passes through the circulator 102 and is split by the splitter/merger 103 into the object light beam R1 and the reference light beam R2. The object light beam R1 is further split by the splitter/merger 104 into the first object light beam R11 and the second object light beam R12. The first object light beam R11 and the second object light beam R12 output from the splitter/merger 104 are radiated onto the measurement target object 200 via the radiation optical system 107. Specifically, the radiation optical system 107 causes the plurality of object light beams R11 and R12 to be radiated to different positions in a plane X-Y in the measurement target object 200, so that a fixed range of the measurement target object 200 is scanned with the object light beams R11 and R12.

The object light beams R11 and R12 radiated onto the measurement target object 200 are scattered backward (in the direction opposite to the radiation direction of the object light beams R11 and R12) by the measurement target object 200. The object light (backscattered light) beams R31 and R32 scattered by the measurement target object 200 pass through the radiation optical system 107, returns to the splitter/merger 104, where the light beams R31 and R32 merge with each other and return to the splitter/merger 103.

The reference light beam R2 output from the splitter/merger 103 is reflected off the reference light beam mirror 108 and returns to the splitter/merger 103.

At the splitter/merger 103, the object light beam R3 generated as a result of the merger of the object light beams R31 and R32 and incident from the splitter/merger 104 interferes with the reference light beam R4 reflected off the reference light beam mirror 108. That is, the splitter/merger 103, at which the object light beams R31 and R32 scattered by the measurement target object 200 interfere with the reference light beam R4 reflected off the reference light beam mirror 108, generates the interference light beams R51 and R52.

The interference light beam R51 is input to the balanced light receiver 109 via the circulator 102, and the interference light beam R52 is directly input to the balanced light receiver 109. Information on the intensity difference between the interference light beams R51 and R52 is then input from the balanced light receiver 109 to the optical spectrum data generation unit 110.

The balanced light receiver 109 then photoelectrically converts an intensity difference $I(k)$ between the interference light beams R51 and R52. The balanced light receiver 109 inputs information on the photoelectrically converted intensity difference $I(k)$ between the interference light beams R51 and R52 to the optical spectrum data generation unit 110.

The optical spectrum data generation unit 110 generates interference light beam spectrum data based on a signal input from the wavelength sweeping laser light source 101 and a signal input from the balanced light receiver 109. Specifically, information on a change in the wavelength of the light emitted from the wavelength sweeping laser light source 101 is input from the wavelength sweeping laser light source 101 to the optical spectrum data generation unit 110. Information on the intensity difference $I(k)$ between the interference light beams R51 and R52 is further input from the balanced light receiver 109 to the optical spectrum data generation unit 110. The optical spectrum data generation unit 110 then generates the interference light beam spectrum data (dependence of the intensity difference $I(k)$ between the interference light beams R51 and R52 on wavelength) based on the information on a change in the wavelength of the light emitted from the wavelength-sweeping laser light source 101 and the information on the intensity difference $I(k)$ between the interference light beams R51 and R52.

The control unit 111 acquires structural data in the depth direction (direction Z) of the measurement target object 200 based on the interference light beam spectrum data generated by the optical spectrum data generation unit 110. The control unit 111 controls the radiation optical system 107 to acquire a plurality of sets of structural data in the depth direction while moving the radiation positions of the plurality of object light beams R11 and R12 along a direction (at least one of directions X and Y) perpendicular to the depth direction (direction Z) of the measurement target object 200. In other words, the control unit 111 acquires a plurality of sets of structural data in the depth direction at different positions along at least one of the directions X and Y of the measurement target object 200. The control unit 111 then connects the plurality of sets of acquired structural data in the depth direction to each other to acquire two-dimensional or three-dimensional tomographic structural data.

The aforementioned optical coherence tomography apparatus 100 according to the present invention, in which the object light beam is split by the splitter/merger 104 into the plurality of object light beams R11 and R12, which are then radiated onto the measurement target object 200, can measure the measurement target object 200 at high speed over a wide range. The object light beams R31 and R32 backscattered by the measurement target object 200 are merged by the splitter/merger 104 to generate the object light beam R3. The splitter/merger 103, at which the object light beam R3 interferes with the reference light beam R4 reflected off the reference light beam mirror 108, generates the interference light beams R51 and R52. There is therefore no need to increase the number of optical paths for the reference light beams R2 and R4 or increase the number of balanced light receivers 109. A compact optical coherence tomography apparatus 100 capable of wide-range, high-speed measurement at low cost can thus be provided.

First example embodiment

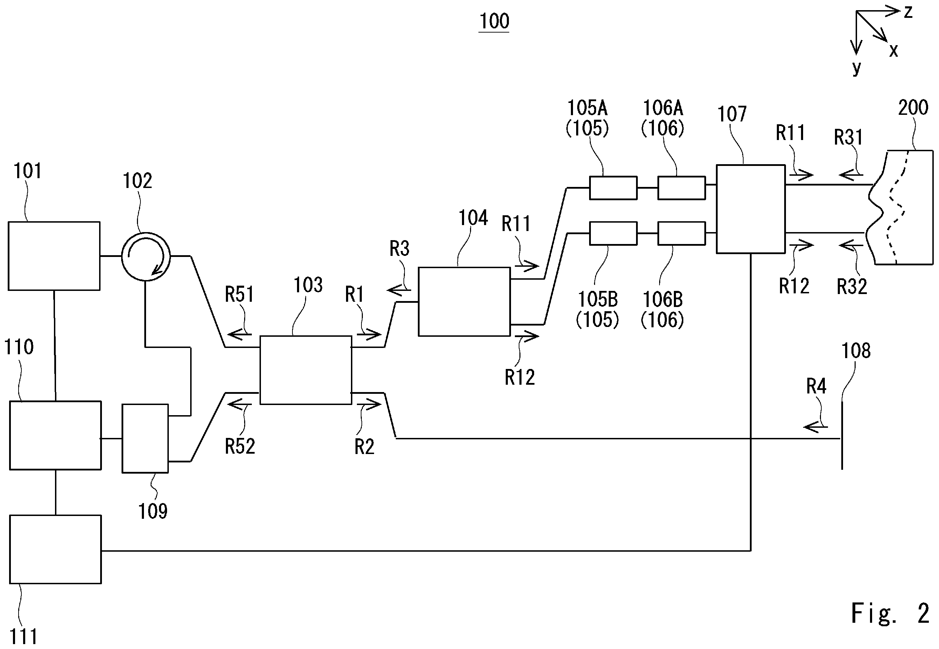

An optical coherence tomography apparatus 100 according to a first example embodiment of the present invention will be described. Fig. 2 shows an example of the optical coherence tomography apparatus 100 according to the first example embodiment. The optical coherence tomography apparatus 100 includes the wavelength sweeping laser light source 101, the circulator 102, the splitter/merger 103, which serves as the first splitter unit and merging unit, the splitter/merger 104, which serves as the second splitter unit, a plurality of optical delayers 105A, 105B, ..., which serve as a delayer unit, (optical delayers 105A, 105B, ... are simply referred to as optical delayers 105 when these optical delayers are not particularly distinguished from each other), a plurality of fiber collimators 106A, 106B, ... (fiber collimators 106A, 106B, ... are simply referred to as fiber collimators 106 when these fiber collimators are not particularly distinguished from each other), the radiation optical system 107, which serves as the radiating unit, the reference light beam mirror 108, the balanced light receiver 109, which serves as the measuring unit, the optical spectrum data generation unit 110, which serves as the measuring unit, the control unit 111, and other components, as shown in Fig. 2.

The number of optical delayers 105 and the number of fiber collimators 106 provided in the optical coherence tomography apparatus 100 may be determined in accordance with the number of split light beams produced at the splitter/merger 104, and are each not limited to the number shown Fig. 2.

The wavelength sweeping laser light source 101 generates wavelength-swept optical pulses. Specifically, the wavelength sweeping laser light source 101 generates optical pulses each having a wavelengths that increases from 1250 nm to 1350 nm during a duration of 10 $\mu$s. The wavelength sweeping laser light source 101 generates the optical pulse repeatedly at 50 kHz every 20 $\mu$s.

The light emitted from the wavelength sweeping laser light source 101 passes through the circulator 102 and is split by the splitter/merger 103 into the object light beam R1 and the reference light beam R2. The object light beam R1 is further split by the splitter/merger 104 into the first object light beam R11 and the second object light beam R12. The first object light beam R11 and the second object light beam R12 output from the splitter/merger 104 are radiated onto the measurement target object 200 via the optical delayers 105, the fiber collimators 106, and the radiation optical system 107. Specifically, the radiation optical system 107 causes the plurality of object light beams R11 and R12 to be radiated to different positions in a plane X-Y in the measurement target object 200, so that a fixed range of the measurement target object 200 is scanned with the object light beams R11 and R12. For example, the radiation optical system 107 uses a raster scan to scan the measurement target object 200.

The object light beams R11 and R12 radiated onto the measurement target object 200 are scattered backward (in the direction opposite to the radiation direction of the object light beams R11 and R12) by the measurement target object 200. The object light (backscattered light) beams R31 and R32 scattered by the measurement target object 200 pass through the radiation optical system 107, the fiber collimators 106, and the optical delayers 105, returns to the splitter/merger 104, where the light beams R31 and R32 merge with each other and further return to the splitter/merger 103.

The reference light beam R2 output from the splitter/merger 103 is reflected off the reference light beam mirror 108 and returns to the splitter/merger 103.

At the splitter/merger 103, the object light beam R3 generated as a result of the merger of the object light beams R31 and R32 and incident from the splitter/merger 104 interferes with the reference light beam R4 reflected off the reference light beam mirror 108. That is, the splitter/merger 103, at which the object light beams R31 and R32 scattered by the measurement target object 200 interfere with the reference light beam R4 reflected off the reference light beam mirror 108, generates the interference light beams R51 and R52.

The interference light beam R51 is input to the corresponding balanced light receiver 109 via the circulator 102, and the interference light beam R52 is directly input the corresponding balanced light receiver 109. The information on the intensity difference between the interference light beams R51 and R52 is then input from the balanced light receiver 109 to the optical spectrum data generation unit 110. The balanced light receiver 109 is, for example, a light receiver in which two photodiodes are connected in series to each other, and an output (a differential output) is generated at the connected point. The band over which the balanced light receiver 109 operates is lower than or equal to 1 GHz.

Consider now the interference between the object light beams R31, R32 and the reference light beam R4 having a wavelength $\lambda$ and a wave number k (=$2\pi/\lambda$). Let $L_R$ be the optical path length of the reference light beam R2 from the point where the reference light beam R2 is split by splitter/merger 103 to the point where the reference light beam R2 is reflected off the reference light beam mirror 108 and returns to the splitter/merger 103. On the other hand, let $L_{S1}=L_R+z_1$ be the optical path length from the point where the first object light beam R11 is split by the splitter/merger 103 to the point where the first object light beam R11 is backscattered at a single light scattering point in the measurement target object 200 and returns to the splitter/merger 103. $L_{S2}=L_R+z_2$ be the optical path length from the point where the second object light beam R12 is split by the splitter/merger 103 to the point where the second object light beam R12 is backscattered at a single light scattering point in the measurement target object 200 and returns to the splitter/merger 103. The relative relationship between $z_1$ and $z_2$ is set by adjusting the optical delayers 105A and 105B. At the splitter/merger 103, the first object light beam R31 and the reference light beam R4 are superimposed on each other with a phase difference $kz_1+\varphi_1$ and interfere with each other. The symbol $\varphi_1$ is a constant independent of k and $z_1$. The second object light beam R32 and the reference light beam R4 are superimposed on each other with a phase difference $kz_2+\varphi_2$ and interfere with each other. The symbol $\varphi_2$ is a constant independent of k and $z_2$. Furthermore, the first object light beam R31 and the second object light beam R32 are superimposed on each other with a phase difference $k(z_1-z_2)+\varphi_3= kz_3+\varphi_3$ and interfere with each other. When $0<z_2<z_1$ is satisfied, $z_3=z_1-z_2$ and $\varphi_3$ are each a constant independent of k and $z_3$.

Let $E_{S1}$ be the amplitude of the object light beam R31, which interferes at the splitter/merger 103, $E_{S2}$ be the amplitude of the object light beam R32, and $E_R$ be the amplitude of the reference light beam R4, and the intensity difference between the interference light beams R51 and R52 is expressed by

[Expression 3]

$$I(k) \propto E_{S1} \cdot E_R \cdot \cos(kz_1 + \phi_1) + E_{S2} \cdot E_R \cdot \cos(kz_2 + \phi_2)$$
$$+ E_{S1} \cdot E_{S2} \cdot \cos(kz_3 + \phi_3)$$

The balanced light receiver 109 then photoelectrically converts an intensity difference I(k) between the interference light beams R51 and R52. The balanced light receiver 109 inputs information on the photoelectrically converted intensity difference I(k) between the interference light beams R51 and R52 to the optical spectrum data generation unit 110.

The optical spectrum data generation unit 110 generates interference light beam spectrum data based on the signal input from the wavelength sweeping laser light source 101 and the signal input from the balanced light receiver 109. Specifically, the information on a change in the wavelength of the light emitted from the wavelength sweeping laser light source 101 is input from the wavelength sweeping laser light source 101 to the optical spectrum data generation unit 110. The information on the intensity difference I(k) between the interference light beams R51 and R52 is further input from the balanced light receiver 109 to the optical spectrum data generation unit 110. The optical spectrum data generation unit 110 then generates the interference light beam spectrum data based on the information on a change in the wavelength of the light emitted from the wavelength-sweeping laser light source 101 and the information on the intensity difference I(k) between the interference light beams R51 and R52. The interference light beam spectral data I(k) generated through the measurement made from the wave number $k_0-\Delta k/2$ to $k_0+\Delta k/2$ shows modulations with cycles $2\pi/z_1$, $2\pi/z_2$, and $2\pi/z_3$ overlapping with each other. The optical spectrum data generation unit 110 inputs the generated interference light beam spectrum I(k) to the control unit 111. Note that $k_0$ satisfies $k_0=2\times\pi/$(wavelength-sweeping light source center wavelength). In the present first example embodiment, the center wavelength of the light from the wavelength sweeping laser 101 is 1300 nm, so that $k_0=2\pi\times7692$ cm$^{-1}$.

The control unit 111 performs Fourier transformation on the interference light beam spectrum data I(k). An amplitude P(z) generated as a result of the Fourier transformation of the interference light beam spectrum data I(k) is expressed by

[Expression 4]

$$P(z) = \left| \int I(k)e^{izk}dk \right|$$

$$\propto a_1\big(\delta(z - z_1) + \delta(z + z_1)\big) + a_2\big(\delta(z - z_2) + \delta(z + z_2)\big)$$
$$+ a_3\big(\delta(z - z_3) + \delta(z + z_3)\big)$$

The amplitude P(z) represents a peak expressed by the $\delta$ function (delta function) (hereafter referred to as "$\delta$-function-like peak") at $z=z_1$ (and $z=-z_1$), which reflects the scattering point position zi, where the first object light beam R31 is scattered, and at $z=z_2$ (and $z=-z_2$), which reflects the scattering point position $z_2$, where the second object light beam R32 is scattered. At the amplitude P(z), the effect of the optical interference between the first object light beam R31 and the second object light beam R32 also appears at $z=z_3$ (and $z=-z_3$). However, the optical intensities of the first object light beam R31 and the second object light beam R32 are smaller than the optical intensity of the reference light beam R4, the component produced by the optical interference between the first object light beam R31 and the second object light beam R32 is small. Symbols $a_1$, $a_2$, and $a_3$ are predetermined constants determined in advance under a variety of conditions.

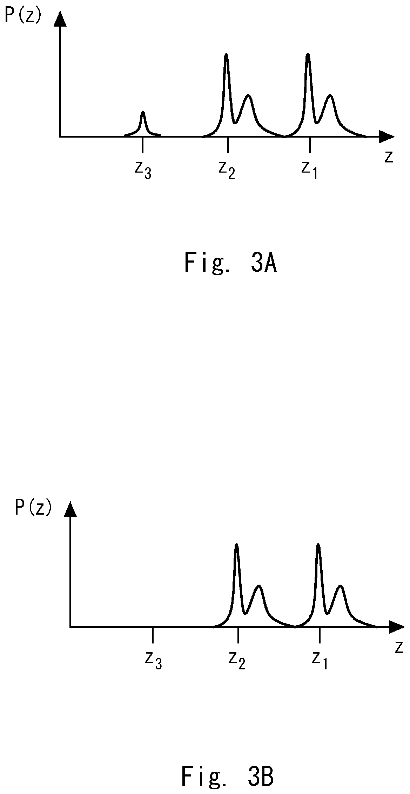

Fig. 3A shows an example of the amplitude P(z) produced as a result of Fourier transformation of the interference light beam spectrum data I(k) when the scattering points of the first object light beam R31 in the measurement target object are distributed over a certain range containing zi, and the scattering points of the second object light beam R32 in the measurement target object are distributed over a certain range containing $z_2$. The control unit 111 performs filtering operation to suppress the modulations in the vicinity of the cycle $2\pi/z_3$ that appears in the interference light beam spectrum I(k) and then performs Fourier transformation. Components in the vicinity of $z_3$ can thus be suppressed, as shown in Fig. 3B. In accordance with the procedure described above, data showing the intensities (amplitudes P(z)) of the plurality of object light beams R31 and R32 backscattered at different positions in the depth direction (direction Z) of the measurement target object 200 can be generated by causing the reference light beam R4 common to the plurality of object light beams R31 and R32 to interfere therewith (operation of generating data representing the intensity of the backscattered light beam (object light beam) in the depth direction (direction Z) at a certain position in the measurement target object 200 is hereafter referred to as "A scan").

The control unit 111 also controls each unit of the optical coherence tomography apparatus 100.

For example, the control unit 111 controls the radiation optical system 107 to cause the plurality of object light beams R11 and R12 to be radiated to different positions in a plane X-Y in the measurement target object 200. The control unit 111 further controls the cycle and speed at which the measurement target object 200 is scanned with the light from the radiation optical system 107.

The control unit 111 further controls the radiation optical system 107 to repeatedly perform the A-scan operation while moving the radiation positions of the object light beams R11 and R12 in the scanning line direction (at least one of the directions X and Y). The control unit 111 then connects a plurality of measurement results generated by repeatedly performing the A-scan operation while moving the radiation positions of the object light beams R11 and R12 in the scanning line direction. The control unit 111 thus generates two-dimensional tomographic structural data (operation of repeatedly performing the A-scan operation in the scanning line direction (at least one of the directions X and Y) and connecting the measurement results to each other is hereafter referred to as "B-scan").

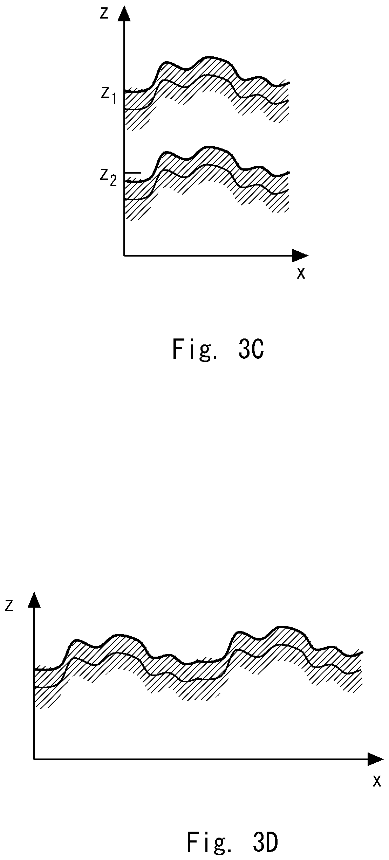

Fig. 3C shows an example of a map of the intensities of the object light beams R31 and R32 backscattered at each position in a plane X-Z in the measurement target object 200 (two-dimensional tomographic structural data). In Fig. 3C, the horizontal axis represents the coordinate X, and the vertical axis represents the coordinate Z. That is, Fig. 3C shows tomographic structural data representing the measurement target object 200 and generated by the B scan. The vicinity of $z_1$ shows the first object light beam R11 (R31) backscattered by the measurement target object 200, and the vicinity of zz shows the second object light beam R12 (R32) backscattered by the measurement target object 200.

Furthermore, the control unit 111 corrects the tomographic structural data in Fig. 3C based on $z_1$ and $z_2$ determined in accordance with the settings of the optical delayers 105A and 105B to connect the tomographic structural data sets to each other which are measured by the plurality of object light beams R11 and R12 radiated to different regions. A wide-range tomographic structural data, such as that shown in Fig. 3D, can thus be generated.

The control unit 111 further controls the radiation optical system 107 to repeatedly perform the B-scan operation while moving the radiation positions of the object light beams R11 and R12 are radiated not only in the scanning line direction but in a direction perpendicular to the scanning line. The control unit 111 then connects a plurality of measurement results to each other, which are generated by repeatedly performing the B-scan operation while moving the radiation positions of the object light beams R11 and R12 in the scanning line direction and the direction perpendicular to the scanning line. The control unit 111 thus generates three-dimensional tomographic structural data in the directions X, Y, and Z (operation of repeatedly performing the B-scan operation in the scanning line direction and a direction perpendicular to the scanning line and connecting the measurement results to each other is hereafter referred to as "C-scan").

The control unit 111 executes the process of connecting a plurality of sets of three-dimensional tomographic structural data to each other, which are generated by sweeping the plurality object light beams R11 and R12.

The control unit 111 includes a CPU (central processing unit) that is not shown, a storage unit that is not shown, and other components. The CPU then executes a program stored in the storage unit to achieve all processes executed by the control unit 111.

The program stored in each storage unit of the control unit 111 contains codes executed by the CPU to achieve the corresponding process executed by the control unit 111. The storage unit includes any storage apparatus capable of, for example, storing the program and a variety pieces of information used in the process executed by the control unit 111. The storage apparatus is, for example, a memory.

The program described above can be stored by using any of a variety of types of non-transitory computer readable medium and supplied to a computer. The non-transitory computer readable medium include a variety of types of tangible storage medium. Examples of the non-transitory computer readable medium include a magnetic storage medium (floppy disk, magnetic tape, and hard disk drive, for example), a magneto-optical storage medium (magneto-optical disk, for example), a CD-ROM (read only memory), CD-R, CD-R/W, and a semiconductor memory (mask ROM, PROM (programmable ROM), an EPROM (erasable PROM), a flash ROM, a RAM (random access memory), for example). The program may instead be supplied to a computer via any of a variety of types of transitory computer readable medium. Examples of the transitory computer readable medium include an electric signal, an optical signal, and an electromagnetic wave. The transitory computer readable medium can supply the program to a computer via a wired communication path, such as an electric wire and an optical fiber, or a wireless communication path.

According to the aforementioned optical coherence tomography apparatus 100 according to the first example embodiment, in which the object light beam is split by the splitter/merger 104 into the plurality of object light beams R11 and R12, which are then radiated onto the measurement target object 200, can measure the measurement target object 200 at high-speed over a wide range. The object light beams R31 and R32 backscattered by the measurement target object 200 are merged by the splitter/merger 104 to generate the object light beam R3. The splitter/merger 103, at which the object light beam R3 interferes with the reference light beam R4 reflected off the reference light beam mirror 108, generates the interference light beams R51 and R52. There is therefore no need to increase the number of optical paths for the reference light beams R2 and R4 or increase the number of balanced light receivers 109. A compact optical coherence tomography apparatus 100, an imaging method, and a non-transitory computer readable medium storing an imaging program each capable of wide-range, high-speed measurement at low cost can thus be provided.

The control unit 111 controls the radiation optical system 107 to acquire a plurality of sets of structural data in the depth direction while moving the radiation positions of the plurality of object light beams R11 and R12 along two directions perpendicular to the depth direction (direction Z) of the measurement target object 200 and perpendicular to each other (directions X and Y). Three-dimensional tomographic structural data can therefore be acquired.

The splitter/merger 103 causes the object light beam R3, which is the merger of the object light beams R31 and R32, to interfere with the reference light beam R4 reflected off the reference light beam mirror 108 to generate the interference light beams R51 and R52. It is therefore unnecessary to add extra components that cause the object light beam R3 and the reference light beam R4 to interfere with each other.

The plurality of optical delayers 105 provided between the splitter/merger 104 and the radiation optical system 107 delay the plurality of object light beams R11 and R12 so as to have different optical path lengths from the point where the plurality of object light beams R11 and R12 are split by the splitter/merger 104 to the point where the plurality of object light beams R11 and R12 are backscattered by the measurement target object 200 and return to the splitter/merger 104. The control unit 111 then corrects the tomographic structural data based on the delays $z_1$ and $z_2$, created by the optical delayers 105, of the optical path lengths of the plurality of object light beams R11 and R12 split by the splitter/merger 104. The control unit 111 can thus connect the plurality of sets of structural data acquired in the depth direction to each other while moving the radiation positions of the plurality of object light beams R11 and R12.

Second example embodiment

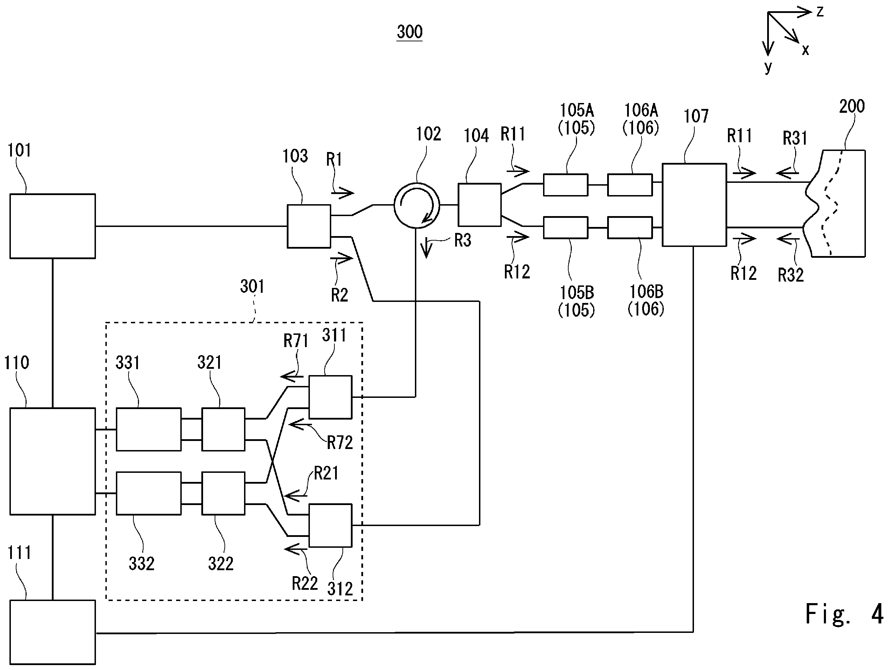

An optical coherence tomography apparatus 300 according to a second example embodiment of the present invention will be described. Fig. 4 shows an example of the optical coherence tomography apparatus 300 according to the second example embodiment. The optical coherence tomography apparatus 300 includes the wavelength sweeping laser light source 101, the splitter/merger 103, which serves as the first splitter unit, the circulator 102, the splitter/merger 104, which serves as the second splitter unit, the plurality of optical delayers 105A, 105B, ... (optical delayers 105A, 105B, ... are simply referred to as optical delayers 105 when these optical delayers are not particularly distinguished from each other), the plurality of fiber collimators 106A, 106B, ... (fiber collimators 106A, 106B, ... are simply referred to as fiber collimators 106 when these fiber collimators are not particularly distinguished from each other), the radiation optical system 107, which serves as the radiating unit, a coherent light receiver 301, the optical spectrum data generation unit 110, which serves as the measuring unit, the control unit 111, and other components, as shown in Fig. 4. The number of optical delayers 105 and the number of fiber collimators 106 provided in the optical coherence tomography apparatus 300 may be determined in accordance with the number of split light beams produced at the splitter/merger 104, and are each not limited to the number shown in Fig. 4.

The wavelength sweeping laser light source 101, the circulator 102, the splitter/merger 103, the splitter/merger 104, the optical delayers 105, the fiber collimators 106, the radiation optical system 107, the optical spectrum data generation unit 110, and the control unit 111 provided in the optical coherence tomography apparatus 300 according to the second example embodiment have the same functions as those of the wavelength sweeping laser light source 101, the circulator 102, the splitter/merger 103, the splitter/merger 104, the optical delayers 105, the fiber collimators 106, the radiation optical system 107, the optical spectrum data generation unit 110, and the control unit 111 provided in the optical coherence tomography apparatus 100 according to the first example embodiment and therefore have the same reference characters, and the same functions will not be described.

The light emitted from the wavelength sweeping laser light source 101 is split by the splitter/merger 103 into the object light beam R1 and the reference light beam R2. The object light beam R1 passes through the circulator 102 and is further split by the splitter/merger 104 into the first object light beam R11 and the second object light beam R12. The first object light beam R11 and the second object light beam R12 output from the splitter/merger 104 are radiated onto the measurement target object 200 via the optical delayers 105, the fiber collimators 106, and the radiation optical system 107. Specifically, the radiation optical system 107 causes the plurality of object light beams R11 and R12 to be radiated to different positions in a plane X-Y in the measurement target object 200, so that a fixed range of the measurement target object 200 is scanned with the object light beams R11 and R12.

The object light beams R11 and R12 radiated onto the measurement target object 200 are scattered backward (in the direction opposite to the radiation direction of the object light beams R11 and R12) by the measurement target object 200. The object light (backscattered light) beams R31 and R32 scattered by the measurement target object 200 pass through the radiation optical system 107, the fiber collimators 106, and the optical delayers 105, returns to the splitter/merger 104, where the light beams R31 and R32 merge with each other, and the merged light beam is guided to the coherent light receiver 301 via the circulator 102.

The reference light beam R2 output from the splitter/merger 103 is guided to the coherent light receiver 301.

At the coherent light receiver 301, a first splitter 311 splits the object light beam R3 generated as a result of the merger of the object light beams R31 and R32 incident from the splitter/merger 104 into object light beams R71 and R72. The object light beams R71 and R72 from the first splitter 311 are guided to a first merger 321 and a second merger 322, respectively. A second splitter 312 splits the reference light beam R2 into the reference light beams R21 and R22. The reference light beams R21 and R22 from the second splitter 312 are guided to the first merger 321 and the second merger 322, respectively. At the first merger 321, the object light beam R71 and the reference light beam R21 interfere with each other, and at the second merger 322, the object light beam R72 and the reference light beam R22 interfere with each other. The optical path length from the second splitter 312 to the first merger 321 and the optical path length from the second splitter 312 to the second merger 322 are so set that the difference therebetween is half the wavelength ($\lambda/2$). The phase difference between the object light beam R71 and the reference light beam R21, which interfere with each other at the first merger 321, and the phase difference between the object light beam R72 and the reference light beam R22, which interfere with each other at the second merger 322, is therefore $\pi$. The first merger 321 inputs the two light beam outputs to a first balanced light receiver 331 (first measurer), and the first balanced light receiver 331 photoelectrically converts the intensity difference between the two light beams. The first balanced light receiver 331 outputs the intensity difference between the photoelectrically converted two light beams to the optical spectrum data generation unit 110. The second merger 322 inputs the two light beam outputs to a second balanced light receiver 332 (second measurer), and the second balanced light receiver 332 photoelectrically converts the intensity difference between the two light beams. The second balanced light receiver 332 outputs the intensity difference between the photoelectrically converted two light beams to the optical spectrum data generation unit 110.

Consider now the interference between the object light beams R71, R72 and the reference light beams R21, R22 having the wavelength $\lambda$ and the wave number k ($=2\pi/\lambda$). Let $L_R$ be the optical path length of the reference light beam R2 from the point where the reference light beam R2 is split by splitter/merger 103 to the point where the reference light beam R2 propagates to the first merger 321. In this case, the optical path length of the reference light beam R2 from the point where the reference light beam R2 is split by splitter/merger 103 to the point where the reference light beam R2 propagates to the second merger 322 is $L_R+(\lambda/2)$. On the other hand, let $L_{S1}=L_R+z_1$ be the optical path length from the point where the first object light beam R11 is split by the splitter/merger 103 to the point where the first object light beam R11 is backscattered at a single light scattering point in the measurement target object 200 and propagates to the first merger 321 or the second merger 322. Let $L_{S2}=L_R+z_2$ be the optical path length from the point where the second object light beam R12 is split by the splitter/merger 103 to the point where the second object light beam R12 is backscattered at a single light scattering point in the measurement target object 200 and propagates to the first merger 321 or the second merger 322. $z_1$ and $z_2$ are set by adjusting the optical delayers 105A and 105B. At the first merger 321, the first object light beam R31 and the reference light beam R2 are superimposed with each other with the phase difference $kz_1+\varphi_1$ and interfere with each other. At the second merger 322, the first object light beam R31 and the reference light beam R2 are superimposed with each other with a phase difference $kz_1+\varphi_1+\pi$ and interfere with each other. The symbol $\varphi_1$ is a constant independent of k and $z_1$. At the first merger 321, the second object light beam R32 and the reference light beam R2 are superimposed with each other with the phase difference $kz_2+\varphi_2$ and interfere with each other. At the second merger 322, the second object light beam R32 and the reference light beam R2 are superimposed with each other with a phase difference $kz_2+\varphi_2+\pi/2$ and interfere with each other. The symbol $\varphi_2$ is a constant independent of k and $z_2$. Furthermore, the first object light beam R31 and the second object light beam R32 are superimposed with each other at the splitter/merger 104 with the phase difference $k(z_1-z_2)+\varphi_3=kz_3+\varphi_3$ and interfere with each other. The symbol $\varphi_3$ is a constant independent of k and $z_3$.

Using the first balanced light receiver 331 and the second balanced light receiver 332 allows detection of a state in which the object light beams R31 and R32 interfere with the reference light beam R2 with a phase difference of $\pi/2$ (quadrature phase). Under the definitions that $E_{S1}$ represents the amplitude of the object light beam R31, $E_{S2}$ represents the amplitude of the object light beam R32, and $E_R$ represents the amplitude of the reference light beam R2, which interfere with each other at the coherent light receiver 301, the optical spectrum data generation unit 110 generates interference light beam spectrum data expressed by

[Expression 5]

$$I(k) \propto E_{S1} \cdot E_R \cdot \{\cos(kz_1 + \phi_1) + i\,\sin(kz_1 + \phi_1)\}$$
$$+ E_{S2} \cdot E_R \cdot \{\cos(kz_2 + \phi_2) + i\,\sin(kz_2 + \phi_2)\}$$
$$+ E_{S1} \cdot E_{S2} \cdot \cos(kz_3 + \phi_3)$$

based on the information on a change in the wavelength of the light emitted from wavelength sweeping laser light source 101 and information on the interference light beam intensity difference measured by each of the first balanced light receiver 331 and the second balanced light receiver 332. The interference light beam spectral data I(k) generated through the measurement made from the wave number $k_0-\Delta k/2$ to $k_0+\Delta k/2$ shows modulations with the cycles $2\pi/z_1$, $2\pi/z_2$, and $2\pi/z_3$ overlapping with each other. The optical spectrum data generation unit 110 inputs the interference light beam spectrum data I(k) to the control unit 111.

The control unit 111 performs Fourier transformation on the interference light beam spectrum data I(k). The amplitude P(z) generated as a result of the Fourier transformation of the interference light beam spectrum data I(k) is expressed by

[Expression 6]

$$P(z) = \left| \int I(k) e^{izk} dk \right|$$

$$\propto a_1 \cdot \delta(z - z_1) + a_2 \cdot \delta(z - z_2) + a_3 \cdot \delta(z - z_3)$$

The amplitude P(z) shows the $\delta$-function-like peak at $z=z_1$, which reflects the scattering point position $z_1$ of the first object light beam R31, and at $z=z_2$, which reflects the scattering point position $z_2$ of the second object light beam R32. At the amplitude P(z), the effect of the optical interference between the first object light beam R31 and the second object light beam R32 also appears at $z=z_3$. However, the optical intensities of the first object light beam R31 and the second object light beam R32 are smaller than the optical intensity of the reference light beam R2, the component produced by the optical interference between the first object light beam R31 and the second object light beam R32 is small.

In the first example embodiment, performing Fourier transformation on the interference light beam spectrum at a single light scattering point provides two $\delta$-function-like peaks at two locations symmetrical with respect to z=0, whereas in the second example embodiment using the coherent light receiver 301, the $\delta$-function-like peak appears at only one location. Therefore, in the first example embodiment, it is necessary to adjust the optical path length of the object light beam in such a way that $z_1$ and $z_2$ are either positive or negative values, whereas in the second example embodiment, it is not necessary that $z_1$ and $z_2$ are limited to either positive or negative values. The range over which the optical path length of the object light beam can be set is thus expanded.

The control unit 111 performs filtering operation to suppress modulation in the vicinity of the cycle $2\pi/z_3$ that appears in the interference light beam spectrum I(k) and then performs Fourier transformation, as in the first example embodiment. Components in the vicinity of $z_3$ can thus be suppressed, as shown in Fig. 3B. According to the procedure described above, data representing the intensities of a plurality of object light beams backscattered at different positions in the depth direction (direction Z) of the measurement target object 200 are generated by interference with a common reference light beam (A scan).

The processes in the B scan and the processes in the C scan performed by the control unit 111 are also the same as those in the first example embodiment and will therefore not described.

The optical coherence tomography apparatus 300 according to the second example embodiment described above provides the same effects as those provided by the optical coherence tomography apparatus 100 according to the first example embodiment. In the coherent light receiver 301, the optical path length from the second splitter 312 to the first merger 321 and the optical path length from the second splitter 312 to the second merger 322 are so set that the difference therebetween is half the wavelength ($\lambda/2$). The phase difference between the object light beam R71 and the reference light beam R21, which interfere with each other at the first merger 321, and the phase difference between the object light beam R72 and the reference light beam R22, which interfere with each other at the second merger 322, therefore differs from each other by $\pi/2$. The amplitude P(z) produced when the control unit 111 performs Fourier transformation on the interference light beam spectrum data I(k) therefore shows the $\delta$-function-like peak at $z=z_1$, which reflects the scattering point position $z_1$ of the first object light beam R31, and at $z=z_2$, which reflects the scattering point position $z_2$ of the second object light beam R32. That is, in the first example embodiment, performing Fourier transformation on the interference light beam spectrum at a single light scattering point provides two $\delta$-function-like peaks at two locations symmetrical with respect to $z=0$, whereas in the second example embodiment, the $\delta$-function-like peak appears at only one location. Therefore, in the first example embodiment, the optical delayers 105 need to adjust the optical path length of the object light beam in such a way that $z_1$ and $z_2$ are either positive or negative values, whereas in the second example embodiment, it is not necessary that $z_1$ and $z_2$ are limited to either positive or negative values. The range over which the optical path length of the object light beam can be set is thus expanded.

Third example embodiment

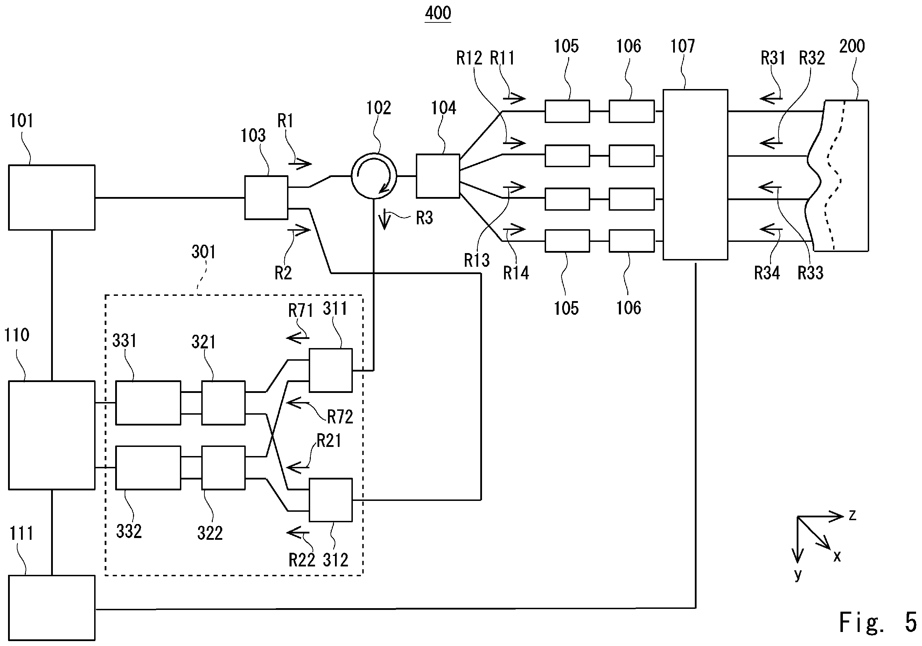

An optical coherence tomography apparatus 400 according to a third example embodiment of the present invention will be described. Fig. 5 shows an example of the optical coherence tomography apparatus 400 according to the third example embodiment. The optical coherence tomography apparatus 400 differs from the optical coherence tomography apparatus 300 according to the second example embodiment in that the object light beam R1 is split into four object light beams R11, R12, R13, and R14 at the splitter/merger 104, as shown in Fig. 5.

The second example embodiment has been described with reference to the configuration in which the two object light beams R11 and R12 are radiated, and the number of object light beams radiated onto the measurement target object 200 is not limited to two. In fact, in the third example embodiment, the reference optical path and the first balanced light receiver 331 and the second balanced light receiver 332 are shared in a configuration in which four object light beams are radiated to different areas of the measurement target object 200, as shown in Fig. 5. Wide-range, high-speed measurement is thus achieved at low cost and in a compact configuration.

Fourth example embodiment

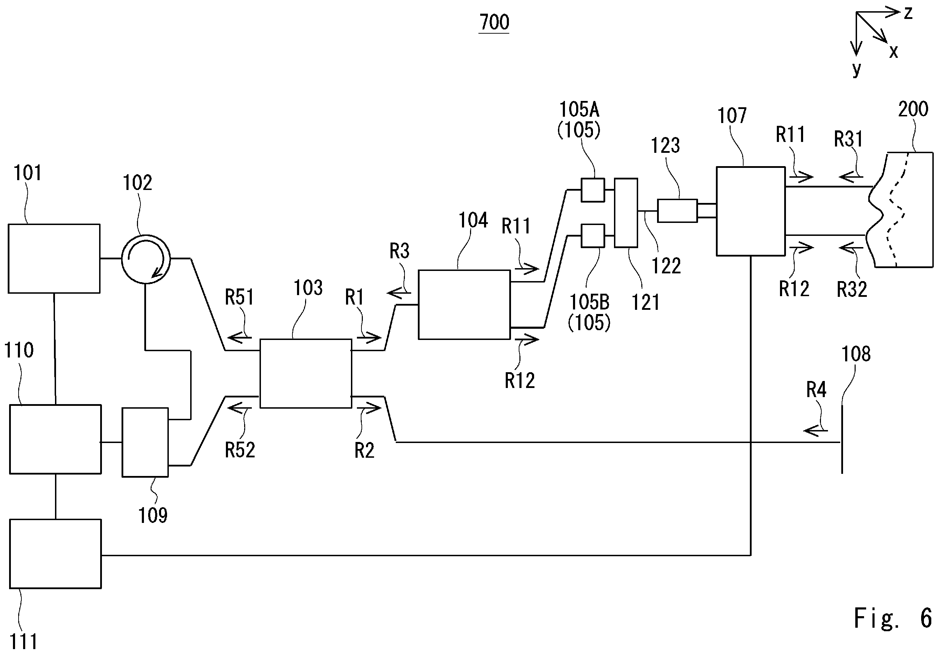

An optical coherence tomography apparatus 700 according to a fourth example embodiment of the present invention will be described. Fig. 6 shows an example of the optical coherence tomography apparatus 700 according to the second example embodiment. The optical coherence tomography apparatus 700 includes the wavelength sweeping laser light source 101, the splitter/merger 103, which serves as the first splitter unit, the circulator 102, the splitter/merger 104, which serves as the second splitter unit, the plurality of optical delayers 105A, 105B, ... (optical delayers 105A, 105B, ... are simply referred to as optical delayers 105 when these optical delayers are not particularly distinguished from each other), an optical connection unit 121 between a plurality of single-mode optical fibers (SMFs) and a single multicore optical fiber (MCF), an MCF 122, a fiber collimator 123, the radiation optical system 107, which serves as the radiating unit, a coherent light receiver 109, the optical spectrum data generation unit 110, which serves as the measuring unit, the control unit 111, and other components, as shown in Fig. 6. The number of optical delayers 105 provided in the optical coherence tomography apparatus 700 may be determined in accordance with the number of split beams produced at the splitter/merger 104, and is not limited to the number shown in Fig. 6.

The wavelength sweeping laser light source 101, the circulator 102, the splitter/merger 103, the splitter/merger 104, the optical delayers 105, the fiber collimators 106, the radiation optical system 107, the optical spectrum data generation unit 110, and the control unit 111 provided in the optical coherence tomography apparatus 700 according to the fourth example embodiment have the same functions as those of the wavelength sweeping laser light source 101, the circulator 102, the splitter/merger 103, the splitter/merger 104, the optical delayers 105, the radiation optical system 107, the optical spectrum data generation unit 110, and the control unit 111 provided in the optical coherence tomography apparatus 100 according to the first example embodiment and therefore have the same reference characters, and the same functions will not be described.

The light emitted from the wavelength sweeping laser light source 101 passes through the circulator 102 and is split by the splitter/merger 103 into the object light beam R1 and the reference light beam R2. The object light beam R1 is further split by the splitter/merger 104 into the first object light beam R11 and the second object light beam R12. The first object light beam R11 and second object light beam R12 output from the splitter/merger 104 are radiated onto the measurement target object 200 via the optical delayers 105, the SMF-MCF connection unit 121, the MCF 122, the fiber collimator 123, and the radiation optical system 107. Specifically, the radiation optical system 107 causes the plurality of object light beams R11 and R12 to be radiated to different positions in a plane X-Y in the measurement target object 200, so that a fixed range of the measurement target object 200 is scanned with the object light beams R11 and R12.

The object light beams R11 and R12 radiated onto the measurement target object 200 are scattered backward (in the direction opposite to the radiation direction of the object light beams R11 and R12) by the measurement target object 200. The object light (backscattered light) beams R31 and R32 scattered by the measurement target object 200 pass through the radiation optical system 107, the fiber collimator 123, the MCF 122, the MCF-SMF connection unit 121, and the optical delayers 105, returns to the splitter/merger 104, where the light beams R31 and R32 merge with each other and return to the splitter/merger 103.

The reference light beam R2 output from the splitter/merger 103 is reflected off the reference light beam mirror 108 and returns to the splitter/merger 103.

At the splitter/merger 103, the object light beam R3 generated as a result of the merger of the object light beams R31 and R32 and incident from the splitter/merger 104 interferes with the reference light beam R4 reflected off the reference light beam mirror 108. That is, the splitter/merger 103, at which the object light beams R31 and R32 scattered by the measurement target object 200 interfere with the reference light beam R4 reflected off the reference light beam mirror 108, generates the interference light beams R51 and R52.

The interference light beam R51 is input to the corresponding balanced light receiver 109 via the circulator 102, and the interference light beam R52 is directly input the corresponding balanced light receiver 109. Information on the intensity difference between the interference light beams R51 and R52 is then input from the balanced light receiver 109 to the optical spectrum data generation unit 110.

The optical spectrum data generation unit 110 generates the interference light beam spectrum data based on the information on a change in the wavelength of the light emitted from the wavelength-sweeping laser light source 101 and the information on the intensity difference I(k) between the interference light beams R51 and R52, as in the first example embodiment. The optical spectrum data generation unit 110 then inputs the generated interference light beam spectrum to the control unit 111.

The control unit 111 performs filtering operation on the interference light beam spectrum and then performs Fourier transformation, as in the first example embodiment. According to the procedure described above, data representing the intensities of a plurality of object light beams backscattered at different positions in the depth direction (direction Z) of the measurement target object 200 are generated by interference with a common reference light beam (A scan).

The processes in the B scan and the processes in the C scan performed by the control unit 111 are also the same as those in the first example embodiment and will therefore not described.

The optical coherence tomography apparatus 700 according to the fourth example embodiment described above provides the same effects as those provided by the optical coherence tomography apparatus 100 according to the first example embodiment. In addition, the MCF 122 and the fiber collimator 123 can be used to configure a compact light radiation unit.

The present application invention has been described above with reference to the embodiments, but the invention of the present application is not limited thereto. A variety of changes can be made to the configurations and details of the invention of the present application to the extent that the changes are understandable to those skilled in the art within the scope of the invention.

## Industrial Applicability

A compact optical coherence tomography apparatus 100, an imaging method, and a non-transitory computer readable medium storing an imaging program each capable of wide-range, high-speed measurement at low cost can be provided.

## Reference Signs List

    100, 300, 400, 700 OPTICAL COHERENCE TOMOGRAPHY APPARATUS
    101 WAVELENGTH SWEEPING LASER LIGHT SOURCE
    102 CIRCULATOR
    103 SPLITTER/MERGER (FIRST SPLITTER UNIT, MERGING UNIT)
    104 SPLITTER/MERGER (SECOND SPLITTER UNIT)
    105A, 105B OPTICAL DELAYER

106A, 106B FIBER COLLIMATOR
107 RADIATION OPTICAL SYSTEM (RADIATING UNIT)
108 REFERENCE LIGHT BEAM MIRROR
109 BALANCED LIGHT RECEIVER (MEASURING UNIT)
110 OPTICAL SPECTRUM DATA GENERATION UNIT (MEASURING UNIT)
111 CONTROL UNIT
121 SMF-MCF CONNECTION UNIT
122 MCF
123 FIBER COLLIMATOR
301 COHERENT LIGHT RECEIVER (MERGING UNIT)
311 FIRST SPLITTER
312 SECOND SPLITTER
321 FIRST MERGER
322 SECOND MERGER
331 FIRST BALANCED LIGHT RECEIVER (FIRST MEASURER)
332 SECOND BALANCED LIGHT RECEIVER (SECOND MEASURER)
R1, R3, R11, R12, R13, R14, R31, R32, R33, R34, R71, R72 OBJECT LIGHT BEAM
R2, R4, R21, R22 REFERENCE LIGHT BEAM
R5, R6, R51, R52, R53 INTERFERENCE LIGHT BEAM
200 MEASUREMENT TARGET OBJECT

**Claims**

1. An optical coherence tomography apparatus comprising:

    a wavelength sweeping laser light source;
    a first splitter unit that splits a light beam emitted from the wavelength sweeping laser light source into an object light beam and a reference light beam;
    a second splitter unit that splits the object light beam output from the first splitter unit into a plurality of object light beams;
    a radiating unit that radiates the plurality of object light beams output from the second splitter unit to different positions on a surface of a measurement target object;
    a merging unit that causes an object light beam to interfere with the reference light beam to generate a plurality of interference light beams, the object light beam generated by merging the plurality of object light beams obtained by radiation onto the measurement target object and then scattering by the measurement target object, with one another at the second splitter unit;
    a measuring unit that generates information on wavelength dependence of an intensity difference among the plurality of interference light beams output from the merging unit; and
    a control unit that acquires structural data in a depth direction of the measurement target object based on the information relating to wavelength dependence of an intensity difference among the plurality of interference beams and generated by the measuring unit, acquires a plurality of sets of structural data in the depth direction while controlling the radiating unit to move radiation positions of the plurality of object light beams along a direction perpendicular to the depth direction of the measurement target object, and connects the plurality of sets of acquired structural data in the depth direction to each other.

2. The optical coherence tomography apparatus according to claim 1,
wherein the control unit acquires a plurality of sets of structural data in the depth direction while controlling the radiating unit to move the radiation positions of the plurality of object light beams along two directions perpendicular to the depth direction of the measurement target object and perpendicular to each other.

3. The optical coherence tomography apparatus according to claim 1 or 2, wherein the merging unit is the first splitter unit.

4. The optical coherence tomography apparatus according to claim 1 or 2,

    wherein the merging unit includes
    a first splitter that splits the object light beam, which is generated by merging the plurality of object light beams obtained by radiation onto the measurement target object and then scattering by the measurement target object, with one another at the second splitter unit, into two object light beams,

a second splitter that splits the reference light beam into two reference light beams,

a first merger that causes one of the two object light beams output from the first splitter to interfere with one of the two reference light beams output from the second splitter to generate two interference light beams,

a second merger that causes another one of the two object light beams output from the first splitter to interfere with another one of the two reference light beams output from the second splitter to generate two interference light beams,

a first measurer that measures an intensity difference between the two interference light beams output from the first merger, and

a second measurer that measures an intensity difference between the two interference light beams output from the second merger,

a difference between an optical path length from the second splitter to the first merger and an optical path length from the second splitter to the second merger is half the wavelength ($\lambda/2$), and

the measuring unit generates information on wavelength dependence of the intensity difference between the interference light beams output from the first measurer and the intensity difference between the interference light beams output from the second measurer.

5. The optical coherence tomography apparatus according to any one of claims 1 to 4,

further comprising a delayer unit that is located between the second splitter unit and the radiating unit and delays the plurality of object light beams in such a way that there are differences among optical path lengths of the plurality of object light beams from a point where the plurality of split object light beams are generated at the second splitter unit to a point where the plurality of object light beams are backscattered in the measurement target object and return to the second splitter unit,

wherein the control unit connects the plurality of sets of structural data in the depth direction acquired while moving the radiation positions of the plurality of object light beams based on the delayed amounts of optical path lengths of the plurality of object light beams.

6. An imaging method comprising:

causing a control unit to, after an object light beam split from a light beam emitted from a wavelength sweeping laser light source is further split into a plurality of object light beams, and the plurality of object light beams are radiated to different positions on a target object, then scattered by the measurement target object, and merged with one another into an object light beam, acquire structural data in a depth direction of a measurement target object based on information on wavelength dependence of an intensity difference among a plurality of interference light beams generated when the object light beam interferes with a reference light beam split from the light beam emitted from the wavelength sweeping laser light source;

causing the control unit to acquire a plurality of sets of structural data in the depth direction while moving radiation positions of the plurality of object light beams along a direction perpendicular to the depth direction of the measurement target object; and

causing the control unit to connect the plurality of sets of acquired structural data in the depth direction to each other.

7. The imaging method according to claim 6, wherein the control unit acquires a plurality of sets of structural data in the depth direction while moving the radiation positions of the plurality of object light beams along two directions perpendicular to the depth direction of the measurement target object and perpendicular to each other.

8. A non-transitory computer readable medium storing an imaging program, the program causing a control unit to

after an object light beam split from a light beam emitted from a wavelength sweeping laser light source is further split into a plurality of object light beams, and the plurality of object light beams are radiated to different positions on a target object, then scattered by the measurement target object, and merged with one another into an object light beam, execute a process of acquiring structural data in a depth direction of a measurement target object based on information on wavelength dependence of an intensity difference among a plurality of interference light beams generated when the object light beam interferes with a reference light beam split from the light beam emitted from the wavelength sweeping laser light source;

a process of acquiring a plurality of sets of structural data in the depth direction while moving radiation positions of the plurality of object light beams along a direction perpendicular to the depth direction of the measurement target object; and

a process of connecting the plurality of sets of acquired structural data in the depth direction to each other.

9. The non-transitory computer readable medium storing the imaging program according to claim 8, wherein the program causes the control unit to execute the process of acquiring a plurality of sets of structural data in the depth direction while moving the radiation positions of the plurality of object light beams along two directions perpendicular to the depth direction of the measurement target object and perpendicular to each other.

Fig. 1

Fig. 2

EP 4 129 156 A1

P(z)

Fig. 3A

$z_3$ $z_2$ $z_1$ z

P(z)

Fig. 3B

$z_3$ $z_2$ $z_1$ z

Fig. 3C

Fig. 3D

Fig. 4

Fig. 5

700

101

102

105A
(105)

123

107

R11    R31

200

R51

103

R1

R3

104

R11

122

121

R12    R32

110

105B
(105)

R12

R2

R52

R4

108

109

111

Fig. 6

EP 4 129 156 A1

Fig. 7

EP 4 129 156 A1

Fig. 8

600

620
R31 R11
R12 R32
605
604 604
R41 606
606 R42
R11 R21 R12 R22
603 603
R51 R52
610
607 607
601
608
609

## INTERNATIONAL SEARCH REPORT

International application No.

PCT/JP2020/013050

**A. CLASSIFICATION OF SUBJECT MATTER**
A61B 3/10(2006.01)i
FI: A61B3/10 100

According to International Patent Classification (IPC) or to both national classification and IPC

**B. FIELDS SEARCHED**

Minimum documentation searched (classification system followed by classification symbols)
A61B3/10, G01N21/17

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2016-505828 A (LEHIGH UNIVERSITY) 25 February 2016 (2016-02-25) paragraphs [0004], [0018]–[0019], [0025]–[0041], fig. 1 | 1-2, 5-9<br>3-4 |
| Y | JP 2017-529196 A (NOVARTIS AG) 05 October 2017 (2017-10-05) fig. 1 | 3 |
| Y | WO 2010/143601 A1 (UNIVERSITY OF TSUKUBA) 16 December 2010 (2010-12-16) fig. 1 | 3 |
| Y | JP 2010-540914 A (CARL ZEISS MEDITEC AG) 24 December 2010 (2010-12-24) paragraph [0031] | 4 |
| A | JP 2017-46924 A (TOMEY CORPORATION) 09 March 2017 (2017-03-09) paragraphs [0024]–[0033] | 1-9 |
| A | JP 2013-113587 A (TOPCON CORPORATION) 10 June 2013 (2013-06-10) claim 1 | 1-9 |

☐ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 23 April 2020 (23.04.2020) | 16 June 2020 (16.06.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**
Information on patent family members

International application No.

PCT/JP2020/013050

| Patent Documents referred in the Report | Publication Date | Patent Family | Publication Date |
|---|---|---|---|
| JP 2016-505828 A | 25 Feb. 2016 | US 2014/0160488 A1 paragraphs [0004], [0035]-[0036], [0042]-[0058] WO 2014/088650 A1 EP 2929288 A1 CN 104854423 A | |
| JP 2017-529196 A | 05 Oct. 2017 | US 2016/0022484 A1 fig. 1 WO 2016/014289 A1 EP 3131457 A1 CN 106659381 A | |
| WO 2010/143601 A1 | 16 Dec. 2010 | US 2012/0120408 A1 fig. 1 | |
| JP 2010-540914 A | 24 Dec. 2010 | US 2010/0284021 A1 paragraph [0032] WO 2009/043557 A1 EP 2193328 A1 CN 101878410 A | |
| JP 2017-46924 A | 09 Mar. 2017 | (Family: none) | |
| JP 2013-113587 A | 10 Jun. 2013 | (Family: none) | |

Form PCT/ISA/210 (patent family annex) (January 2015)

**REFERENCES CITED IN THE DESCRIPTION**

**Patent documents cited in the description**

- JP 2010167253 A

- WO 2006054116 A